# EUROPEAN PATENT SPECIFICATION

(11) **EP 2 021 037 B1**
(45) Date of publication and mention of the grant of the patent: **01.09.2010**
(21) Application number: 07776870.3
(22) Date of filing: 08.05.2007
(51) Int. Cl.: A61L 2/18, A61L 2/28, A61L 101/22

(54) **PROCESS FOR THE PREPARATION OF A READY-TO-USE DISINFECTANT**
VERFAHREN ZUR HERSTELLUNG EINES VERWENDUNGSFERTIGEN DESINFEKTIONSMITTELS
MÉTHODE DE PRÉPARATION D'UN DÉSINFECTANT PRÊT À L'EMPLOI

(30) Priority: 09.05.2006 US 798907 P; 17.04.2007 US 787731
(43) Date of publication of application: 11.02.2009
(73) Proprietor: E.I. DU PONT DE NEMOURS AND COMPANY, Wilmington, DE 19898 (US)
(72) Inventor: AMES, Kelly, Ann, Sudbury Suffolk CO10 2BL (GB)
(74) Representative: DuPont Performance Coatings Biering/Blum/Kimpel
(86) International application number: PCT/US2007/011085
(87) International publication number: WO 2007/133532

(56) References cited:
- WO-A-97/19594
- WO-A-98/20094
- US-A- 3 982 892
- US-A- 4 110 242
- US-A- 5 021 182
- US-A1- 2004 040 860
- US-A1- 2006 030 505

## Description

The invention relates to a process for the preparation of a ready-to-use disinfectant of the aqueous peracid (peroxycarboxylic acid) type, to the ready-to-use disinfectant and to a disinfection process making use of the ready-to-use disinfectant.

### Background of the Invention

The term "ready-to-use disinfectant" used in the description and the claims means the disinfectant at a concentration at which the disinfectant is applied by the user.

Disinfectants containing compounds that fluoresce on UV (ultraviolet) irradiation as a monitoring means are known from WO 98/21569 and WO 98/20094. Such disinfectants allow for determining whether they have been used correctly in terms of completeness of application, i.e., whether they have or have not reached all parts of a substrate surface to be disinfected during application by simply irradiating the substrate surface with UV light during or after application of the disinfectant and observing the fluorescence by the human eye.

It would be desirable to use the monitoring principle described in the preceding paragraph also in disinfectants of the aqueous peracid type.

Disinfectant compositions comprising a peracid are for instance known from WO 97/19 984.

However, the simple addition of a compound capable of showing fluorescence on UV irradiation to disinfectants of the aqueous peracid type does not yield disinfectants with a satisfactory and sufficiently sustainable fluorescence property. The compounds capable of showing fluorescence on UV irradiation undergo fast decomposition or chemical change in the presence of the aggressive biocides contained in such disinfectants, for example, a peracid, a combination of peracid and hydrogen peroxide or a combination of peracid, the corresponding carboxylic acid and hydrogen peroxide; i.e., the disinfectant loses its fluorescence property rapidly which is unacceptable from the user's standpoint.

### Summary of the Invention

It has now been found that it is possible to prepare disinfectants of the aqueous peracid type having the required sustainable fluorescence property, i.e., a fluorescence which is stable over the working life of the disinfectant, if a first aqueous composition comprising peracid is mixed with a second composition comprising an organic compound capable of showing fluorescence on UV irradiation and, preferably, also a surfactant.

Accordingly, the present invention is related to a process for the preparation and application of an aqueous ready-to-use disinfectant composition as defined in claim 1.

### Detailed Description of the Invention

The process according to the invention involves a process for the preparation of an aqueous ready-to-use disinfectant composition of the so-called aqueous equilibrium peracid type, i.e., disinfectants on the basis of an aqueous composition, preferably an aqueous solution, comprising as essential constituents hydrogen peroxide, at least one peracid and the corresponding one or more carboxylic acids present in a chemical equilibrium. The term "the corresponding one or more carboxylic acids" means the carboxylic acid(s) corresponding to the one or more specified peracids. The process comprises the following successive steps:
1) (a) providing an aqueous preparation A comprising, in a chemical equilibrium with each other, hydrogen peroxide, at least one peracid and the corresponding at least one carboxylic acid; and (b) providing a preparation B comprising at least one organic compound capable of showing fluorescence on UV irradiation and, preferably, at least one surfactant C; and
2) mixing the preparations A and B and, optionally, water, wherein the composition of the preparations A3 and B and the mixing ratio used in step 2) is selected in such a manner that the resultant aqueous ready-to-use disinfectant composition comprises
   0.005 to 1 wt.%, preferably 0.01 to 0.5 wt.% of the at least one peracid,
   0.006 to 1.2 wt.%, preferably 0.012 to 0.6 wt.% of the corresponding at least one carboxylic acid,
   0.025 to 5 wt.%, preferably 0.05 to 2.5 wt.% of hydrogen peroxide,
   0.0001 to 3 wt.% of the at least one organic compound capable of showing fluorescence on UV irradiation and, preferably,
   0.001 to 30 wt.% of the at least one surfactant C.

The peracids used in the process of the invention are C1-C3 peracids, peracetic acid being most preferred. Examples of suitable peracids include performic acid, peracetic acid, perpropionic acid and halogen-substituted peracetic acids, such as, for example, monochloroperacetic acid, dichloroperacetic acid, trichloroperacetic acid and trifluoroperacetic acid; the halogen-free peracids being preferred not least because of environmentally friendliness and biodegradability after the ready-to-use disinfectant's use. Accordingly, formic acid, acetic acid, propionic acid and halogen-substituted acetic acids, such as, for example, monochloroacetic acid, dichloroacetic acid, trichloroacetic acid and trifluoroacetic acid are examples of the corresponding carboxylic acids.

In step 1) of the process according to the invention an aqueous preparation A and a preparation B are provided.

In the process according to the invention the aqueous preparations A are in the form of an aqueous equilibrium peracid solution comprising hydrogen peroxide, at least one peracid and the corresponding at least one carboxylic acid.

Aqueous equilibrium peracid solutions and the preparation thereof are well-known, for example, from US 5,489,706, WO 94/20424, US 5,545,374 and US 5,965,033. The aqueous equilibrium peracid solutions may be prepared by mixing a carboxylic acid with hydrogen peroxide and letting the mixture react in aqueous medium. Preferably the preparation happens by mixing hydrogen peroxide to an aqueous solution of the carboxylic acid and letting the mixture react under the catalytic action of a strong acid, such as, for example, sulfuric acid at temperatures of less than 25°C. After the chemical equilibrium has been reached the aqueous equilibrium peracid solution obtained can be stored at temperatures preferably not exceeding 25°C, for example, between 10 and 25°C.

The composition of the aqueous preparations A can vary in wide ranges, depending amongst others on the ratio chosen between hydrogen peroxide, water and the at least one carboxylic acid for the preparation of the respective aqueous equilibrium peracid solutions. For example, the aqueous preparations A may exhibit a weight ratio of 3 to 10 pbw (parts by weight) of hydrogen peroxide : 0.15 to 2.5 pbw of the at least one peracid : 1 pbw of the corresponding at least one carboxylic acid. According to the invention, however, aqueous preparations A comprise 40 to 80 wt.% of water, 14 to 50 wt.% of hydrogen peroxide, 1 to 17 wt.% of the at least one peracid and 0.1 to 17 wt.% of the corresponding at least one carboxylic acid. One example of a commercially available aqueous equilibrium peracetic acid solution that can be used as an aqueous preparation A is the product Hyperox® from DuPont Animal Health Solutions which comprises about 60 wt.% water, 25 wt.% hydrogen peroxide, 6 wt.% acetic acid and 5 wt.% peracetic acid.

The aqueous preparations A may comprise small amounts of strong acid, for example, up to 3 wt.% of a mineral acid, such as, sulfuric acid, which may have served as a catalyst during the preparation of the aqueous equilibrium peracid solution.

The aqueous preparations A may comprise one or more additives. Examples of possible additives are those conventional in disinfectants of the aqueous peracid type, including peroxide decomposition stabilizers, such as, transition metal sequestering (complexing, chelating) agents; surfactants; water hardness stabilizers, for example, such compounds as are mentioned in U.S. 6,254,801 B1; buffers; pH-adjusting components, such as alkaline inorganic salts; viscosity modifiers, for example, thickeners; co-biocides; corrosion inhibitors; builders; catalysts; fragrances and dyes.

The preparations B comprise at least one organic compound capable of showing fluorescence on UV irradiation, preferably in combination with at least one surfactant C. The preparations B may be non-aqueous. However, preferably they are aqueous compositions, for example, solutions, dispersions or emulsions; aqueous solutions B being particularly preferred. If the preparations B do not comprise at least one surfactant C, they are aqueous solutions of the at least one organic compound capable of showing fluorescence on UV irradiation. If the preparations B are non-aqueous, they comprise at least the at least one organic compound capable of showing fluorescence on UV irradiation and the at least one surfactant C. Preferred aqueous preparations B comprise, for example, 40 to 99 wt.% of water, 1 to 50 wt.% of at least one surfactant C and 0.001 to 10 wt.% of at least one organic compound capable of showing fluorescence on UV irradiation.

The at least one organic compound capable of showing fluorescence on UV irradiation present in the preparations B may be selected from various classes of organic substances. The term "organic compound capable of showing fluorescence on UV irradiation" means an organic compound that is capable of generating intense fluorescence under UV irradiation; it shall not be understood to exclude such organic compounds that comprise an inorganic element or moiety in the molecule. For example, salts consisting of an organic ion and an inorganic counterion are expressly not excluded. The at least one organic compound capable of showing fluorescence on UV irradiation may be selected, for example, from among those organic substances conventional as optical brighteners (fluorescent whitening agents) in detergents for laundry applications. An example of a preferred organic compound capable of showing fluorescence on UV irradiation that can be used in preparation B is sodium distyryl biphenylsulfonate which is commercially available as Tinopal® CBS-X from Ciba.

As already mentioned, the preparation B preferably comprises at least one surfactant C. In general the aqueous preparations A do not comprise surfactants C, although they may optionally comprise other surfactants which are different from the at least one surfactant C. However, in the preferred case of preparation B, which comprises at least one surfactant C, the at least one surfactant C may be present in the aqueous preparation A as well, although it is preferred that only preparation B comprises the at least one surfactant C. The at least one surfactant C may be selected from conventional anionic, nonionic and/or amphoteric surfactants. The at least one surfactant C enables for a more homogenous fluorescence on UV irradiation and for avoiding ambiguous monitoring results under UV irradiation. It is preferred to use so-called high-foaming surfactants as surfactants C. To elucidate the high-foaming nature of a surfactant C the following controlled test is performed: A control solution is prepared by adding such amount of Hostapur® SAS 60 (sodium C14-C17 secondary alkylsulphonate from Clariant) to a surfactant-free aqueous equilibrium peracid solution comprising 0.25 wt.% hydrogen peroxide, 0.06 wt.% acetic acid and 0.05 wt.% peracetic acid that a concentration of 0.006 wt.% of sodium C14-C17 secondary alkylsulphonate, calculated as 100% substance, in the control solution is achieved. 30 ml of this control solution is added to a 100 ml measuring cylinder I and stoppered. The measuring cylinder I is inverted 10 times and the volume of foam is measured immediately, after five minutes, and after one hour. In a second experiment 0.03 wt.% of surfactant C is added to the control solution and 30 ml of the resulting mixture are added to a measuring cylinder II similar to measuring cylinder I. The stoppered measuring cylinder II is inverted 10 times and the volume of foam is measured immediately, after five minutes, and after one hour. Surfactants C are classified as surfactants of the high-foaming type if the foam volume in measuring cylinder II is at least 100 % higher than the foam volume in measuring cylinder I, the foam volume in each case being measured one hour after the 10 times inversion. Examples of useful high-foaming surfactants C comprise Berol® DGR-81 from Akzo Nobel (95 wt.% active substance: mixture of a C9-C11 alcohol ethoxylate and an alkyl glucoside) and Mackam® CBS-50G from McIntyre Group, Ltd. (active substance cocamidopropyl hydroxysultaine, 40 wt.%).

The preparations B may comprise one or more further additives. Examples of further possible additives are transition metal sequestering agents, corrosion inhibitors, stabilizers, viscosity modifiers, builders, dyes and fragrances.

In step 2) of the process according to the invention the preparations A and B and water, are mixed, for example, under stirring.

In principle any mixing sequence is possible. To this end, the composition of both of the preparations A and B and the relative ratio thereof needs to be selected accordingly. If at least one of the preparations A and B is a concentrate, the proportion of water to be mixed with the preparations A and/or B needs to be selected accordingly as well. In principle, it is also possible to mix concentrates of preparations A and B and then to dilute with water. If preparation A is a concentrate with a high peracid content of, for example, above 1 to 5 wt.%, it is expedient not to delay the water dilution step and to perform the dilution with water soon or preferably immediately, for example, within 60 minutes after having mixed preparations A and B. However, to achieve best results in terms of sustainability of the fluorescence property, it is preferred to contact the preparations A and B under conditions that allow for the at least one organic compound capable of showing fluorescence on UV irradiation to come into contact with the peracid only at a certain minimum degree of dilution. Preferably the concentration of the at least one peracid is less than 3 wt.% in the aqueous preparation A prior to contacting it with the at least one organic compound capable of showing fluorescence on UV irradiation, i.e., prior to mixing it with the concentrated or already aqueously diluted preparation B; here, it is self-explanatory that the resultant ready-to-use disinfectant composition will have a content of the at least one peracid of less than 3 wt.%, for example, of 0.005 to 1 wt.%. In other words, it is preferred to predilute at least aqueous preparation A with water prior to mixing it with preparation B. The preparations A and B may be prediluted with water to exactly such degree that on mixing them the ready-to-use disinfectant is obtained with the desired composition and without a need of adjusting its concentration by further dilution with water. An also preferred mixing alternative is the simultaneous addition of relatively concentrated preparations A and B in the appropriate ratio into water while stirring the mixture thus ensuring the preferred effect of dilution directly from the start of dosing the preparations A and B into the water.

If step 2) of the process according to the invention is performed in the preferred manner, i.e., including the use of water for dilution purposes, it is preferred to use pure, deionized or distilled water. It is also possible to use tap water, but in this case it is recommended that at least one of the preparations A and B comprises at least one transition metal sequestering agent.

Furthermore, to achieve best results in terms of sustainability of the fluorescence property, the process of the invention includes the step to apply the ready-to-use disinfectant composition obtained in step 2) of the process according to the invention within 48 hours, preferably within 24 hours, of its preparation, i.e., calculated after completion of the mixing step 2). In other words, it is contemplated to store the aqueous preparations A separate from preparations B until mixing step 2) is performed, which happens no longer than 48 hours, more preferred no longer than 24 hours and most preferred just before application of the ready-to-use disinfectant.

This invention further provides a disinfection method which comprises using a ready-to-use disinfectant prepared according to the process described hereinabove. The ready-to-use disinfectant is reliably effective against a large number of germs, in particular pathogenic germs including bacteria, viruses, fungi, spores, yeasts and algae. It may be used for different disinfecting purposes, for example, in the food, milk, brewing or beverage industry; in farming, for example, cattle or poultry breeding, dairy farming, in laying batteries; in the medical or surgery sector; in sanitary hygiene. It may be used in the disinfection of water-circulating systems, but in particular, is used by applying to surfaces for surface disinfection applications, for example, the disinfection of installations; equipment; pipework; containers; bottles; sanitary objects; work surfaces; furniture; walls; floors; ceilings or complete rooms or buildings; shoes and protective clothing of staff; transportation vehicles, especially the wheels thereof. Contact of the ready-to-use disinfectant with the skin or mucous membranes should be avoided. Those skilled in the art will appreciate the need for appropriate safety use of the ready-to-use disinfectant. For the purposes of surface disinfection the ready-to-use disinfectant may be applied by various application methods which are selected dependent on the kind of surface which is to be disinfected. Application methods include fogging (spraying, atomization), wiping, brushing, dipping and rinsing to name only the most common methods. In certain cases the application of the ready-to-use disinfectant may be followed by a water-rinse after the disinfectant has taken effect; however, generally this is not the case.

Depending on the specific disinfection task to be performed the degree of dilution of the ready-to-use disinfectant will be selected at the lower, the upper or between the lower and the upper end of the concentration range of the at least one peracid.

For example, for routine disinfection preparation A may be diluted and mixed with preparation B so that the final ready-to-use disinfectant comprises 0.02 to 0.03 wt.% of peracid and 0.001 to 0.1 wt.% of organic compound capable of showing fluorescence on UV irradiation. Such ready-to-use disinfectant may be applied to a pre-cleaned surface, for example, at a rate of 300ml/m² of surface area by conventional means, for example, using a knapsack sprayer or a pressure washer set.

For example, for fogging disinfection preparation A may be diluted and mixed with preparation B so that the final ready-to-use disinfectant comprises 0.4 to 0.6 wt.% of peracid and 0.001 to 0.1 wt.% of organic compound capable of showing fluorescence on UV irradiation. Such ready-to-use disinfectant may be applied by conventional means, for example, using a thermal fogging machine at a rate of, for example, 17 ml/m³.

For example, for equipment disinfection preparation A may be diluted and mixed with preparation B so that the final ready-to-use disinfectant comprises 0.04 to 0.06 wt.% of peracid and 0.001 to 0.1 wt.% of organic compound capable of showing fluorescence on UV irradiation. The equipment to be disinfected may be immersed in the ready-to-use disinfectant and may or may not be rinsed after removal.

During and/or after application of the ready-to-use disinfectant, preferably before the disinfectant has dried, for example, within 15 minutes, the surface to which the disinfectant has been applied may be irradiated with UV light for monitoring purposes. The UV irradiation enables the user to determine whether the disinfectant has been thoroughly applied as required by the observation of, or the lack of, fluorescence. Conventional sources of UV light in the wavelength range of 280 to 420 nm, such as, for example, optionally doped high, medium and low pressure mercury vapor lamps and gas discharge tubes, such as, for example, low pressure xenon lamps may be used for irradiation of the surfaces with UV light. Generally, the fluorescence is sufficiently bright in the daylight to be observed by the human eye. However, observation of the fluorescence may be supported by taking darkening measures.

### EXAMPLES

pbw means parts by weight.

### Materials used:

Preparation A(i): Hyperox® from DuPont Animal Health Solutions (aqueous equilibrium peracetic acid solution comprising 25 wt.% hydrogen peroxide, 6 wt.% acetic acid and 5 wt.% peracetic acid).

Preparation A(ii): Proxitane® 5 from Solvay Chemicals, Inc. (aqueous equilibrium peracetic acid solution comprising 20 wt.% hydrogen peroxide, 10 wt.% acetic acid and 5 wt.% peracetic acid).

Preparation B(i): Mixture of 5.2 wt.% Tinopal® CBS-X, 5.2 wt.% Ampholak® YCE from Akzo Nobel (sodium cocopropylenediamine tripropionate), 11.3 wt.% Berol® DGR-81 and 78.3 wt.% water.

Preparation B(ii): Mixture of 4.62 wt.% Tinopal® CBS-X, 26.0 wt.% Mackam® CBS-50G and 69.38 wt.% water.

### Example 1

1 wt.% solutions of preparations A(i) or A(ii) were prepared by dilution of preparations A(i) or A(ii) with distilled water. 0.1 pbw of preparation B(i) or 0.113 pbw of preparation B(ii) were added to 100 pbw of the 1 wt.% solutions of preparations A(i) or A(ii) with stirring. Any of the four so prepared final ready-to-use disinfectants comprised 0.05 wt.% peracetic acid and 0.005 wt.% Tinopal® CBS-X.

Immediately, one hour, 24, 48, 72, 96 hours and 7 days after their preparation, the ready-to-use disinfectants were sprayed onto a nonporous black plastic surface using a spray bottle. One spray of each ready-to-use disinfectant was applied. The surface was irradiated with a mercury vapor UV lamp (maximum of the emission spectrum at 302 nm) from a distance of 20 cm. The lighting conditions of the room were bright (daylight conditions, no darkening measures were taken).

The solutions showed visually intense fluorescence on exposure to UV light even 7 days after their preparation.

### Example 2

10 pbw of preparation B(i) or 11.3 pbw of preparation B(ii) were added to 100 pbw of preparations A(i) or A(ii) under stirring. Any of the four so prepared mixtures were immediately diluted with distilled water to obtain final ready-to-use disinfectants comprising 0.05 wt.% peracetic acid and 0.005 wt.% Tinopal® CBS-X.

The ready-to-use disinfectants were tested according to the procedure described in Example 1.

The ready-to-use disinfectants showed visually intense fluorescence on exposure to UV light even 7 days after their preparation.

### Example 3

Example 2 was repeated without performing dilution with distilled water. The results of the visual assessment of the fluorescence are shown in Table 1.

### Example 4

3 wt.% solutions of preparations A(i) or A(ii) were prepared by dilution with distilled water. 10 pbw of preparation B(i) or 11.3 pbw of preparation B(ii) were added to 100 pbw of the 3 wt.% solutions of preparations A(i) or A(ii), with stirring.

The mixtures were tested according to the procedure described in Example 1. The results of the visual assessment of the fluorescence are shown in Table 1.

### Example 5

2 wt.% solutions of preparations A(i) or A(ii) were prepared by dilution with distilled water. 10 pbw of preparation B(i) or 11.3 pbw of preparation B(ii) were added to 100 pbw of the 2 wt.% solutions of preparations A(i) or A(ii), with stirring.

The mixtures were tested according to the procedure described in Example 1. The results of the visual assessment of the fluorescence are shown in Table 1.

### Example 6

10 pbw of preparation B(i) or 11.3 pbw of preparation B(ii) were added to 100 pbw of preparations A(i) or A(ii) under stirring. The four so prepared mixtures were diluted with distilled water at times of 0, 1, 6, 24, 48, and 72 hours after initial preparation. The ratio between the mixtures and the distilled water was the same as in Example 2.

Immediately after their preparation the so prepared ready-to-use disinfectants were tested according to the procedure described in Example 1. The results of the visual assessment of the fluorescence are shown in Table 1.

**Table 1**

| | Time (hours) | Fluorescence Strength (Example 3) | Fluorescence Strength (Example 4) | Fluorescence Strength (Example 5) | Fluorescence Strength (Example 6) |
|---|---|---|---|---|---|
| A(i) + B(i) | 0 | Very strong | Very strong | Very strong | Very strong |
| | 1 | Very strong | Very strong | Very strong | Very strong |
| | 6 | Very strong | Very strong | Very strong | Faint |
| | 24 | Barely visible | Barely visible | Strong | No visible fluorescence |
| | 48 | No visible fluorescence | | | |
| A(i) + B(ii) | 0 | Very strong | Very strong | Very strong | Very strong |
| | 1 | Very strong | Very strong | Very strong | Strong |
| | 6 | Barely visible | Strong | Strong | Barely visible |
| | 24 | No visible fluorescence | No visible fluorescence | No visible fluorescence | No visible fluorescence |
| A(ii) + B(i) | 0 | Very strong | Very strong | Very strong | Very strong |
| | 1 | Very strong | Very strong | Very strong | Very strong |
| | 6 | Very strong | Very strong | Very strong | Very strong |
| | 24 | Very strong | Very strong | Very strong | Strong |
| | 48 | Very strong | Very strong | Very strong | No visible fluorescence |
| | 72 | Faint | Strong | Very strong | |
| | 96 | Barely visible | Quite Strong | Strong | |
| A(ii) + B(ii) | 0 | Very strong | Very strong | Very strong | Very strong |
| | 1 | Very strong | Very strong | Very strong | Very strong |
| | 6 | Very strong | Very strong | Very strong | Strong |
| | 24 | Faint | Very strong | Very strong | No visible fluorescence |
| | 48 | No visible fluorescence | No visible fluorescence | Strong | |
| | 72 | | | Strong | |
| | 96 | | | Faint | |

Table 1 shows the disinfectants of the invention, which comprise a peracid, have sufficiently sustainable fluorescence, which decreases in fluorescence strength over time. In particular, Table 1 shows the influence of concentration and dilution conditions on the rate of decrease of fluorescence strength.

## Claims

1. A process for the preparation and application of an aqueous ready-to-use disinfectant composition comprising the following successive steps:
1) (a) providing an aqueous preparation A in the form of an aqueous equilibrium C1-C3 peracid solution prepared by mixing a C1-C3 carboxylic acid with hydrogen peroxide and letting the mixture react in aqueous medium, the aqueous equilibrium C1-C3 peracid solution comprising, in a chemical equilibrium with each other, hydrogen peroxide, a C1-C3 peracid and the corresponding C1-C3 carboxylic acid; and (b) providing a preparation B comprising at least one organic compound capable of showing fluorescence on UV irradiation; and
2) mixing the preparations A and B and water,
wherein the aqueous preparation A comprises 40 to 80 wt.% of water, 14 to 50 wt.% of hydrogen peroxide, 1 to 17 wt.% of the C1-C3 peracid and 0.1 to 17 wt.% of the corresponding C1-C3 carboxylic acid,
wherein the composition of the preparations A and B and the mixing ratio used in step 2) is selected in such a manner that the resultant aqueous ready-to-use disinfectant composition comprises
0.005 to 1 wt.% of the C1-C3 peracid,
0.006 to 1.2 wt.% of the corresponding C1-C3 carboxylic acid, 0.025 to 5 wt.% of hydrogen peroxide and
0.0001 to 3 wt.% of the at least one organic compound capable of showing fluorescence on UV irradiation,
3) application of the composition, and
wherein mixing step 2) is performed no longer than 48 hours before application of the ready-to-use disinfectant composition.

2. The process of claim 1, wherein preparation A has a C1-C3 peracid content of above 1 to 5 wt.%, and wherein the dilution with water in step 2) is performed within 60 minutes after having mixed preparations A and B.

3. The process of claim 1, wherein the concentration of the C1-C3 peracid is less than 3 wt.% within the aqueous preparation A prior to contacting it with the at least one organic compound capable of showing fluorescence on UV irradiation.

4. The process of any one of the preceding claims, wherein preparation B is an aqueous preparation.

5. The process of any one of the preceding claims, wherein preparation B comprises at least one surfactant C and wherein the resultant ready-to-use disinfectant composition comprises 0.001 to 30 wt.% of the at least one surfactant C.

6. The process of claim 5, wherein the at least one surfactant C is a high-foaming surfactant.

7. The process of any one of the preceding claims, wherein the C1-C3 peracid is peracetic acid.

8. A disinfection method, wherein a ready-to-use disinfectant composition is first prepared according to a process of any one of the preceding claims and then is applied to a surface to be disinfected within 48 hours after completion of the mixing step 2) of said process, and wherein during and/or after application of the ready-to-use disinfectant composition the surface to which the ready-to-use disinfectant composition has been applied is irradiated with UV light for monitoring purposes.

## Patentansprüche

1. Verfahren zur Herstellung und Anwendung einer wässrigen verwendungsfertigen Desinfektionsmittelzusammensetzung, das die folgenden aufeinanderfolgenden Schritte umfasst:
1) (a) das Bereitstellen einer wässrigen Zubereitung A in Form einer wässrigen Äquilibrium-C1-C3-Persäurelösung, die durch Mischen einer C1-C3-Carbonsäure mit Wasserstoffperoxid und Reagierenlassen der Mischung in einem wässrigen Medium hergestellt wird, wobei die wässrige Äquilibrium-C1-C3-Persäurelösung Wasserstoffperoxid, eine C1-C3-Persäure und die entsprechende C1-C3-Carbonsäure in einem chemischen Äquilibrium miteinander umfasst; und (b) das Bereitstellen einer Zubereitung B umfassend mindestens eine organische Verbindung, die dazu fähig ist, beim UV-Bestrahlen Fluoreszenz aufzuweisen; und
2) das Mischen der Zubereitungen A und B und von Wasser,
wobei die wässrige Zubereitung A 40 bis 80 Gew.-% Wasser, 14 bis 50 Gew.-% Wasserstoffperoxid, 1 bis 17 Gew.-% der C1-C3-Persäure und 0,1 bis 17 Gew.-% der entsprechenden C1-C3-Carbonsäure umfasst,
wobei die Zusammensetzung der Zubereitungen A und B und das in Schritt 2) angewendete Mischverhältnis auf derartige Weise ausgewählt werden, dass die so gebildete wässrige verwendungsfertige Desinfektionsmittelzusammensetzung Folgendes umfasst:
0,005 bis 1 Gew.-% der C1-C3-Persäure,
0,006 bis 1,2 Gew.-% der entsprechenden C1-C3-Carbonsäure,
0,025 bis 5 Gew.-% Wasserstoffperoxid und
0,0001 bis 3 Gew.-% der mindestens einen organischen Verbindung, die dazu fähig ist, beim UV-Bestrahlen Fluoreszenz aufzuweisen,
3) das Anwenden der Zusammensetzung und
wobei der Mischschritt 2) für nicht länger als 48 Stunden vor der Anwendung der verwendungsfertigen Desinfektionsmittelzusammensetzung durchgeführt wird.

2. Verfahren nach Anspruch 1, wobei die Zubereitung A einen C1-C3-Persäuregehalt von mehr als 1 bis 5 Gew.-% aufweist und wobei die Verdünnung mit Wasser in Schritt 2) innerhalb von 60 Minuten nach dem Mischen der Zubereitungen A und B durchgeführt wird.

3. Verfahren nach Anspruch 1, wobei die Konzentration der C1-C3-Persäure innerhalb der wässrigen Zubereitung A vor dem Kontaktieren derselben mit der mindestens einen organischen Verbindung, die dazu fähig ist, beim UV-Bestrahlen Fluoreszenz aufzuweisen, weniger als 3 Gew.-% beträgt.

4. Verfahren nach einem der vorhergehenden Ansprüche, wobei die Zubereitung B eine wässrigen Zubereitung ist.

5. Verfahren nach einem der vorhergehenden Ansprüche, wobei die Zubereitung B mindestens ein Tensid C umfasst und wobei die so gebildete verwendungsfertige Desinfektionsmittelzusammensetzung 0,001 bis 30 Gew.-% des mindestens einen Tensids C umfasst.

6. Verfahren nach Anspruch 5, wobei das mindestens eine Tensid C ein stark schäumendes Tensid ist.

7. Verfahren nach einem der vorhergehenden Ansprüche, wobei die C1-C3-Persäure Peressigsäure ist.

8. Desinfektionsmethode, wobei die verwendungsfertige Desinfektionsmittelzusammensetzung zuerst einem Verfahren nach einem der vorhergehenden Ansprüche gemäß zubereitet und dann innerhalb von 48 Stunden nach Abschluss des Mischschritts 2) des Verfahrens auf eine zu desinfizierende Oberfläche aufgebracht wird und wobei während des Aufbringens und/oder nach dem Aufbringen der verwendungsfertigen Desinfektionsmittelzusammensetzung die Oberfläche, auf die die verwendungsfertige Desinfektionsmittelzusammensetzung aufgebracht worden ist, zu Überwachungszwecken mit UV-Licht bestrahlt wird.

## Revendications

1. Procédé pour la préparation et l'application d'une composition aqueuse de désinfectant prêt à l'emploi comprenant les étapes successives suivantes consistant:
1) (a) à fournir une préparation aqueuse A sous la forme d'une solution aqueuse de peracide C1-C3 à l'équilibre préparée en mélangeant un acide carboxylique C1-C3 avec du peroxyde d'hydrogène et à laisser le mélange réagir dans un milieu aqueux, la solution aqueuse de peracide C1-C3 à l'équilibre comprenant, dans un équilibre chimique les uns avec les autres, du peroxyde d'hydrogène, un peracide C1-C3 et l'acide carboxylique C1-C3 correspondant; et (b) à fournir une préparation B comprenant au moins un composé organique capable de montrer une fluorescence lors d'une irradiation UV; et
2) à mélanger les préparations A et B et de l'eau,
dans lequel la préparation aqueuse A comprend de 40 à 80% en poids d'eau, de 14 à 50% en poids de peroxyde d'hydrogène, de 1 à 17% en poids du peracide C1-C3 et de 0,1 à 17% en poids de l'acide carboxylique C1-C3 correspondant,
dans lequel la composition des préparations A et B et le rapport de mélange utilisé dans l'étape 2) sont choisis de manière à ce que la composition aqueuse de désinfectant prêt à l'emploi comprend:
de 0,005 à 1 % en poids du peracide C1-C3,
de 0,006 à 1,2% en poids de l'acide carboxylique C1-C3 correspondant,
de 0,025 à 5% en poids de peroxyde d'hydrogène, et
de 0,0001 à 3% en poids du au moins un composé organique capable de montrer une fluorescence lors d'une irradiation UV,
3) à appliquer la composition, et
dans lequel l'étape de mélange 2) est effectuée pas plus de 48 heures avant l'application de la composition de désinfectant prêt à l'emploi.

2. Procédé selon la revendication 1, dans lequel la préparation A possède une teneur en peracide C1-C3 au-dessus de 1 à 5% en poids et dans lequel la dilution avec de l'eau dans l'étape 2) est effectuée dans les 60 minutes après avoir mélangé les préparations A et B.

3. Procédé selon la revendication 1, dans lequel la concentration du peracide C1-C3 est inférieure à 3% en poids dans la préparation aqueuse A avant sa mise en contact avec le au moins un composé organique capable de montrer une fluorescence lors d'une irradiation UV.

4. Procédé selon l'une quelconque des revendications précédentes, dans lequel la préparation B est une préparation aqueuse.

5. Procédé selon l'une quelconque des revendications précédentes, dans lequel la préparation B comprend au moins un tensioactif C et dans lequel la composition de désinfectant prêt à l'emploi résultante comprend de 0,001 à 30% en poids du au moins un tensioactif C.

6. Procédé selon la revendication 5, dans lequel le au moins un tensioactif C est un tensioactif hautement moussant.

7. Procédé selon l'une quelconque des revendications précédentes, dans lequel le peracide C1-C3 est l'acide peracétique.

8. Procédé de désinfection, dans lequel une composition de désinfectant prêt à l'emploi est tout d'abord préparée conformément à un procédé selon l'une quelconque des revendications précédentes et ensuite la composition est appliquée sur une surface à désinfecter dans les 48 heures après l'achèvement de l'étape de mélange 2) dudit procédé, et dans lequel pendant et/ou après l'application de la composition de désinfectant prêt à l'emploi la surface sur laquelle la composition de désinfectant prêt à l'emploi a été appliquée est irradiée avec une lumière UV à des fins de surveillance.
